(19) Europäisches Patentamt
European Patent Office
Office européen des brevets

(11) Publication number: 0 091 796
A1

(12) **EUROPEAN PATENT APPLICATION**

(21) Application number: 83301977.1

(22) Date of filing: 07.04.83

(51) Int. Cl.[3]: C 07 D 235/30
C 07 D 409/02
//(C07D409/02, 235/30, 333/22)

(30) Priority: 08.04.82 US 366759

(43) Date of publication of application: 19.10.83 Bulletin 83/42

(84) Designated Contracting States: BE CH DE FR GB IT LI LU NL SE

(71) Applicant: ELI LILLY AND COMPANY
307, East McCarty Street
Indianapolis Indiana 46285(US)

(72) Inventor: Morwick, Tina Marie
6811 South Meridian
Indianapolis Indiana 46217(US)

(72) Inventor: Wikel, James Howard
4068 Sunshine Way
Greenwood Indiana 46142(US)

(74) Representative: Crowther, Terence Roger et al,
Erl Wood Manor
Windlesham Surrey GU20 6PH(GB)

(54) **Process for removing 1-sulfonyl groups from benzimidazoles.**

(57) This invention concerns a process for removing a sulfonyl group from the 1-position of a benzimidazole to allow conversion of 2-amino-1,5-substituted-benzimidazole compounds into 2-amino-1,6-substituted-benzimidazole compounds. The 5-isomer is reacted with an alkali metal hydroxide or carbonate in an inert aqueous organic solvent system. The reaction mixture is acidified to precipitate the base and form the intermediate benzimidazole tautomer. The intermediate is then reacted with a sulfonyl acylating agent or a haloalkyl isothiocyanate to form a mixture of 2-amino-1,5(6)-substituted-benzimidazole compounds. This invention also concerns the process for removing a sulfonyl group from the 1-position of a benzimidazole to allow conversion of 2-amino-1,6-substituted-benzimidazole compounds into 2-amino-1,5-substituted-benzimidazole compounds.

EP 0 091 796 A1

Croydon Printing Company Ltd.

# PROCESS FOR REMOVING 1-SULFONYL GROUPS FROM BENZIMIDAZOLES

This invention concerns a process for removing a sulfonyl group from the 1-position of a benzimidazole to allow conversion of the 5-isomer into the 6-isomer and also conversion of the 6-isomer into the 5-isomer.

2-Amino-1,5(6)-substituted-benzimidazole compounds inhibit the growth of certain viruses, such as rhinoviruses, polio (types I, II, III), Coxsackie (A9, A21, B5), echo virus (strains 1, 2, 3, 4), and Mengo virus. Although both the 5- and 6-isomers are useful as antiviral agents, the 6-substituted-2-aminobenzimidazole is generally the more active isomer.

Certain sulfonylbenzimidazole compounds are disclosed in U.S. Patents 4,118,742 and 4,174,454. The preparation of these reference compounds follows the methods disclosed in U.S. Patents 4,018,790 and 4,118,573. Both of these patents reveal the reaction of a benzimidazole compound and a sulfonyl chloride compound in an organic solvent in the presence of a base.

Thiazolinyl and thiazinyl benzimidazole compounds are prepared by the reaction of a 1-unsubstituted-benzimidazole with a haloalkyl isothiocyanate in an organic solvent in the presence of a base, as described in U.S. Patents 4,008,243 and 4,150,028. The references also discuss the compounds use as antiviral agents.

Charles Price and Robert Reitsema in "Some Sulfonamide Derivatives of 2-Aminobenzimidazole," Journal of Organic Chemistry, 12, 269-274 (1947) describe the formation of 2-aminobenzimidazole-3-nitrobenzenesulfonate by treating 1-(3-nitrophenylsulfonyl)-2-aminobenzimidazole with sodium hydroxide and acetic acid.

This invention concerns a process for removing a sulfonyl group from the 1-position of a benzimidazole to allow conversion of 2-amino-1,5-substituted-benzimidazole compounds into a mixture of 5- and 6-substituted isomers, from which the preferred 6-isomer is recovered. The 5-isomer also formed then can be recycled through this process to form more 5- and 6-isomers. This invention also discloses the same process for removing a sulfonyl group from the 1-position of a benzimidazole to allow conversion of the 6-isomer into the 5-isomer.

This process concerns preparing a 2-amino-5(6)-substituted-benzimidazole of the formula

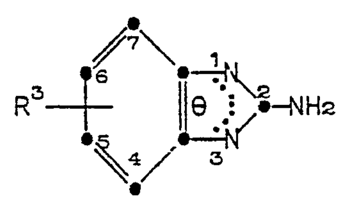 $M^+$

I

wherein:

$R^3$ is $C_1$-$C_4$ alkyl, $C_1$-$C_4$ alkoxy, halo, nitro, $C_1$-$C_4$ alkylthio, phenylthio, phenoxy, carboxy, methylsulfonyl, trifluoromethyl, 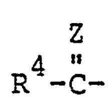 , or 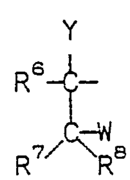 ;

$R^4$ is hydrogen, $C_1$-$C_7$ alkyl, $C_3$-$C_7$ cycloalkyl, ($C_3$-$C_7$ cycloalkyl)methyl, 1-($C_3$-$C_7$ cycloalkyl)ethyl, thienyl, benzyl, phenyl, or mono-substituted phenyl wherein said phenyl substituent is $C_1$-$C_4$ alkyl, $C_1$-$C_4$ alkoxy, chloro, bromo, iodo, nitro, or trifluoromethyl;

$R^6$ is hydrogen, $C_1$-$C_7$ alkyl, $C_3$-$C_7$ cycloalkyl, ($C_3$-$C_7$ cycloalkyl)methyl, 1-($C_3$-$C_7$ cycloalkyl)-ethyl, phenyl, or mono-substituted phenyl, wherein said phenyl substituent is $C_1$-$C_4$ alkyl, $C_1$-$C_4$ alkoxy, chloro, bromo, iodo, nitro, or trifluoromethyl;

$R^7$ and $R^8$ independently are hydrogen, halo, cyano, hydroxymethyl, nitro, $\overset{(O)_m}{S}$ - $C_1$-$C_4$ alkyl, $CH_2R^9$, $COR^9$, phenyl or mono-substituted phenyl, wherein said phenyl substituent is $C_1$-$C_4$ alkyl, $C_1$-$C_4$ alkoxy, chloro, bromo, iodo, nitro, or trifluoromethyl;

$R^9$ is hydroxy, $C_1$-$C_4$ alkoxy, halo, $C_3$-$C_6$ cycloalkyl-$C_1$-$C_4$ alkoxy, or (O-$C_1$-$C_4$ alkyl$)_p$ $NR^{10}R^{11}$ wherein

$R^{10}$ and $R^{11}$ independently are hydrogen or $C_1$-$C_4$ alkyl;

Z is oxygen, $C_1$-$C_7$ alkylidene, or $C_1$-$C_4$ alkoxyimino;

Y is hydrogen, and W is hydroxy, or together Y and W form a bond;

m is 0, 1, or 2;

p is 0 or 1;

$M^+$ is an alkali metal cation or a hydronium ion;

which comprises reacting a 1-sulfonyl-2-amino-5(6)-substituted-benzimidazole of the formula

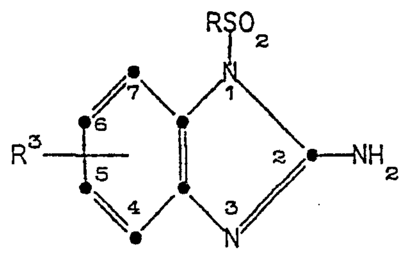

II

wherein:

$R^3$ is defined as above;

R is $C_1$-$C_5$ alkyl, $C_3$-$C_7$ cycloalkyl, phenyl, furyl, or thienyl;

with an alkali metal hydroxide or carbonate, in an inert aqueous organic solvent system, at a temperature from about 20°C to about 100°C.

The 5- and 6-isomer starting material is represented by formula II above.

A 1-substituted-2-amino-5-substituted-benzimidazole of formula

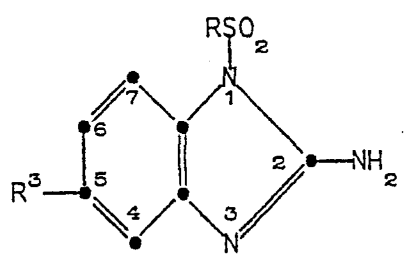

IIA

wherein R and $R^3$ are defined as before;

is reacted by the above process using an alkali metal hydroxide or carbonate in an inert aqueous organic solvent system to form a tautomeric benzimidazole of formula

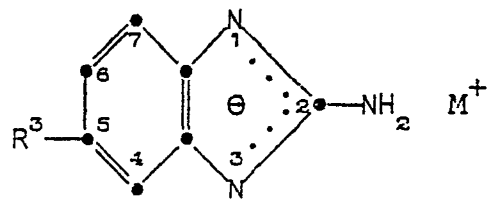 IA

wherein $R^3$ and $M^+$ are defined as before.

When desired, the tautomer IA then can be reacted with a sulfonyl halide of the formula $R^1SO_2X$; or a haloalkyl isothiocyanate of the formula $X(CH_2)_nNCS$, optionally substituted on the carbon chain with $R^5$; to form a mixture of 5- and 6-isomers. This process is described in our copending application . (Agents reference No. X-5091), a copy of which is enclosed herewith. The compounds of formula I above are therefore useful as intermediates.

The desired 2-amino-1,6-substituted-benzimidazole is of formula

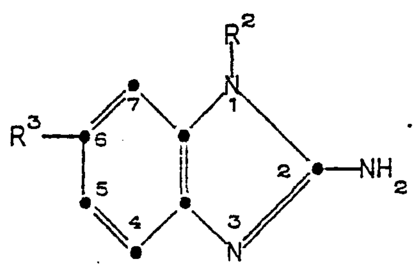 III

wherein $R^3$ is defined as before; and

$R^2$ is $R^1SO_2$ or

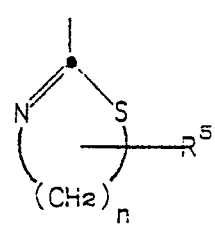

wherein n is defined as before;

$R^1$ is $C_1$-$C_5$ alkyl, $C_3$-$C_7$ cycloalkyl, phenyl, furyl, or thienyl;

$R^5$ is hydrogen, $C_1$-$C_3$ alkyl, benzyl, or phenyl.

Thus, the compounds of formula III are formed from the products of formula I.

A 1-substituted-2-amino-6-substituted-benzimidazole of formula

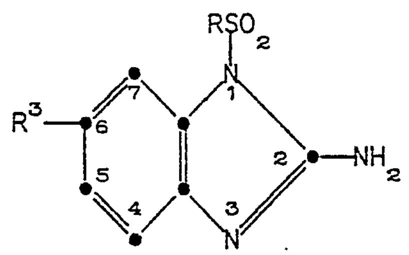

IIB

wherein R and $R^3$ are defined as before; is reacted by the process of this invention using an alkali metal hydroxide or carbonate in an inert aqueous organic solvent system to form a tautomeric benzimidazole of formula

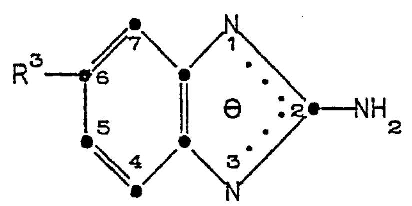

$M^+$ IB

wherein $R^3$ and $M^+$ are defined as before.

The tautomer IB then can be reacted, in a manner analogous to that for formula IA, with a sulfonyl halide of the formula $R^1SO_2X$; or a haloalkyl isothiocyanate of the formula $X(CH_2)_nNCS$, optionally substituted on the carbon chain with $R^5$; to form a mixture of 5- and 6-isomers.

The desired 2-amino-1,5-substituted-benzimidazole is of formula

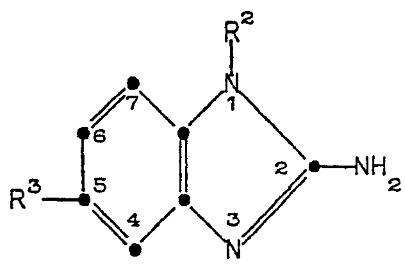

IV

wherein $R^2$ and $R^3$ are defined as before.

The following definitions refer to the various terms used throughout this disclosure. The term "furyl" refers to the furan radical attached at the alpha or beta position. The term "thienyl" refers to the thiophene radical attached at the 2- or 3-position.

The term "thiazolinyl" or thiazolin-2-yl refers to the 4,5-dihydrothiazole radical attached at the 2-position, which may have a substituent group ($R^5$) at the 4- or 5- positions. The term "thiazinyl" or thiazin-2-yl refers to 5,6-dihydro-4H-1,3-thiazine radical attached at the 2-position, which may have a substituent group ($R^5$) at the 4-, 5-, or 6- positions. The substituent group ($R^5$) can be methyl, ethyl, propyl, benzyl, or phenyl.

The term "$C_1$-$C_7$ alkyl" refers to the straight and branched aliphatic radicals of one to seven carbon atoms including methyl, ethyl, propyl, isopropyl, butyl, isobutyl, sec-butyl, tert-butyl, pentyl, isopentyl, sec-pentyl, 1,2-dimethylpropyl, 1,1-dimethylpropyl, neopentyl, hexyl, isohexyl (4-methylpentyl), sec-hexyl (1-methylpentyl), 2-methylpentyl, 3-methylpentyl, 1,1-dimethylbutyl, 2,2-dimethylbutyl, 3,3-dimethylbutyl, 1,2-dimethylbutyl, 1,3-dimethylbutyl, 1,2,2-trimethylpropyl, 1,1,2-trimethylpropyl, heptyl, isoheptyl (5-methylhexyl), sec-heptyl (1-methylhexyl), 1-ethylpentyl, 2,2-dimethylpentyl, 3,3-dimethylpentyl, 4,4-dimethylpentyl, 1,2-dimethylpentyl, 1,4-dimethylpentyl, 1,2,3-trimethylbutyl, 1,1,2-trimethylbutyl, 1,1,3-trimethylbutyl, and others. The term $C_1$-$C_7$ alkyl includes within its definition the terms $C_1$-$C_3$ alkyl, $C_1$-$C_4$ alkyl, and $C_1$-$C_5$ alkyl.

The term "$C_3$-$C_7$ cycloalkyl" refers to the saturated alicyclic rings of three to seven carbon atoms, such as cyclopropyl, methylcyclopropyl, cyclobutyl, cyclopentyl, cyclohexyl, 1-, 2-, 3-, or 4-methylcyclohexyl, and cycloheptyl.

The term "$C_1$-$C_4$ alkoxy" refers to the alkyl radicals of one to four carbon atoms attached to the remainder of the molecule by oxygen and includes methoxy, ethoxy, propoxy, isopropoxy and butoxy.

The term "$\overset{(O)_m}{\overset{|}{S}}$—$C_1$—$C_4$ alkyl" refers to alkyl radicals of one to four carbon atoms attached to a thio, sulfinyl, or sulfonyl group. Examples of such groups include methylthio, ethylthio, isobutylthio, ethylsulfinyl, isobutylsulfinyl, isopropylsulfonyl, <u>n</u>-butylsulfonyl, and <u>tert</u>-butylsulfonyl.

The term "$C_1$-$C_7$ alkylidene" refers to divalent organic radicals of one to seven carbon atoms derived from the corresponding aliphatic hydrocarbons in which two hydrogen atoms are taken from the same carbon atom including ethylidene, propylidene, butylidene, pentylidene, and others.

The terms "halo" and "halide" refer to chloro, bromo, chloride, and bromide.

The term "alkali metal hydroxide or carbonate" refers to sodium or potassium hydroxide, sodium or potassium carbonate, and the like. The base of choice is sodium hydroxide. Any amount of base can be used; but for maximum yield one equivalent of base for every equivalent of benzimidazole is preferred.

The symbol "$M^+$" refers to a hydronium ion or an alkali metal cation, which is the cation of the inorganic base used to remove the sulfonyl group at the 1-position. Therefore, $M^+$ is usually a sodium or potassium cation and is preferably sodium.

The inert aqueous organic solvent system is a combination of water and a water-miscible solvent. The ratio of water to solvent should be from about 10/90 to about 75/25. A nonhydroxylic solvent (one that does not contain hydroxyl groups) is required unless the benzimidazole anion is isolated before sulfonation. If the benzimidazole is isolated, then hydroxylic solvents, such as carbinols, can be used.

Suitable nonhydroxylic solvents include acetone, dimethoxyethane (glyme, DME), tetrahydrofuran (THF), tertiary amides, such as N,N-dimethylformamide (DMF), and the like. The preferred solvent is acetone. Any mixture of the above solvents may be used in place of a single solvent and the addition of an immiscible solvent is permitted as long as the overall solvent combination is water-miscible.

The reaction should be carried out at a temperature from about 20°C to about 100°C, preferably at the reflux temperature of the solvent system employed.

The process can be stopped at the formation of the tautomer of formula I. To form the benzimidazole tautomer, the 5- or 6-isomer is reacted with an alkali metal hydroxide or carbonate in an inert aqueous organic solvent system. Then it can be isolated. If greater than 1 equivalent of hydroxide or carbonate was used to remove the sulfonyl group, the solution containing the benzimidazole tautomer must be acidified to isolate its free base. Obviously, when the tautomer of formula I has been formed, which nitrogen atom is designated as the 1-position is an equal choice. Thus, the 5 and 6 position of formula I are interchangeable.

After the sulfonyl group is removed leaving the benzimidazole anion, a substituent then can be added when desired at the 1-position to form a mixture of 1,5- and 1,6-substituted-benzimidazole compounds. Thus the compounds of

formula I can serve as intermediates. The new substituent at the 1-position can be added by two methods.

The first method is shown in U.S. Patent 4,008,243.

The second method of adding a substituent at the 1-position of the benzimidazole anion is described in our copending application, (Agents reference No. X-5091), a copy of which is enclosed herewith.

The product of the addition reaction is a mixture of 1,5- and 1,6-substituted-benzimidazole compounds, which can be separated and isolated. In those instances where the product precipitates, it may be separated from the reaction mixture by filtration. Alternatively, the reaction mixture may be concentrated to induce crystallization. Or the reaction mixture can be evaporated to dryness and the residue dissolved in a suitable solvent (such as acetone or methanol) to separate and remove any insoluble material. Then the acetone or methanol solution containing the substituted benzimidazole compound is concentrated to crystallize the product or it is evaporated to give a second residue. The second residue is dissolved in a solvent, such as methanol, and the benzimidazole compound is recovered from the solvent by crystallization.

The addition process usually provides about a 1:1 mixture of 1,5- and 1,6-substituted-benzimidazole isomers. The isomers can be separated by fractional

crystallization or by column chromatography. The 6-isomer usually crystallizes first from a solution of the two isomers. The isomers can be identified by their nuclear magnetic resonance spectra in the phenyl proton region (7.0 to 8.3 ppm).

It should be noted that when $R^7$ and $R^8$ in formula I are different, the compounds formed exist as cis and trans isomers. The cis and trans isomers of the benzimidazoles provided herein are represented by the general formulas:

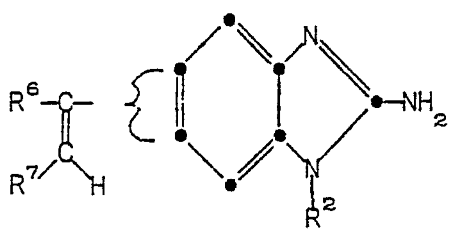

trans isomer

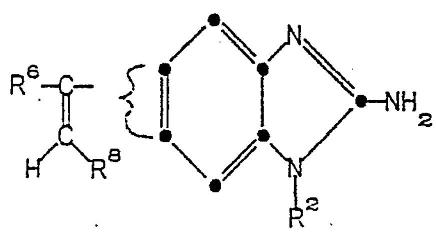

cis isomer

Since both the cis and the trans benzimidazoles are potent antiviral agents, they can be utilized in the treatment of viral infections either alone or as a mixture.

Isolation of pure cis and pure trans benzimidazoles generally is accomplished by chromatography or by crystallization or fractional crystallization from solvent such as methanol, ethanol, acetone, or the like. The trans (E) isomers appear slightly more active than the cis (Z) compounds, and therefore are preferred over the cis (Z) isomers.

The collected 5- or 6-isomer can be recycled through the claimed removal process, if the substituent at the 1-position is a sulfonyl group. Thiazinyl and thiazolinyl groups cannot be removed by this process, and therefore, cannot be recycled.

The preparation of the benzimidazole and sulfonyl halide starting materials for use in the disclosed process is taught in U.S. Patents 4,018,790 and 4,118,573. The preparation of haloalkyl isothiocyanates is taught in U.S. Patent No. 4,008,243.

The following examples are illustrative of this invention, but are not to be considered as limitations on it.

Example 1

2-amino-5(6)-methoximephenylbenzimidazole

A slurry of 5.6 g (0.015 moles) of 2-amino-6-methoximephenyl-1-isopropylsulfonylbenzimidazole was heated on a steam bath in 50 ml of acetone and 50 ml of water with 16 ml of 1 N sodium hydroxide. The heat was continued to remove the acetone by boiling. An oil developed that would not crystallize, so acetone was added to dissolve the oil in the reaction media. The product was precipitated with water, then filtered, and then washed with water. The yield of product was 3.5 g or 88%. There were the expected mass spectrum ions at m/e = 266, and 235, while the $pK_a$ was 8.88. (66% $DMF/H_2O$).

The NMR indicated that two isomers were present as indicated by methoxy resonances.

The following elemental analysis was obtained:
Calculated for $C_{15}H_{14}N_4O \cdot 7/4\ H_2O$

Theory: C, 60.60; N, 5.72; N, 18.85.
Found: C, 60.51; H, 5.42; N, 18.64.

Example 2

2-amino-5(6)-(ethylidenebenzyl)benzimidazole

A slurry of 4.6 g (0.015 moles) of 2-amino-1-isopropylsulfonyl-6-(ethylidenebenzyl)benzimidazole was heated in 50 ml of acetone and 50 ml of water with 16 ml of 1 N sodium hydroxide on a steam bath. The heating was continued to remove the acetone. Afterwards the solution was cooled to room temperature with stirring. When the cleavage was complete, the precipitate title compound was filtered and washed with water.

The yield was 2.75 g (74%). The mass spectrum showed the expected molecular ion at m/e = 249, 172 (loss of -phenyl), 115 (representing -benzimidazole ring). pH 9.06, $pK_a$ 6.98 (66% DMF/$H_2O$).

The following elemental analysis was obtained:
Calculated for $C_{16}H_{15}N_3$:

Theory: C, 77.08; H, 6.06; N, 16.85.
Found: C, 77.33; H, 6.12; N, 16.57.

NMR (DMSO)

| δ | H's | Multiplicity | Group |
|---|---|---|---|
| 1.66 }<br>1.83 } | 3 | doublet<br>doublet | $=CH-CH_3$ |
| 6.0 | 1 | doubled quartet | $=CH-CH_3$ |
| 6.6-7.6 | 8<br>2 | aromatic<br>NH multiplet | |

Example 3

trans-2-amino-5(6)-(ethylidenebenzyl)benzimidazole

A slurry of 1 g (0.0028 moles) of trans-2-amino-1-isopropylsulfonyl-6-(ethylidenebenzyl)benzimidazole was heated in 10 ml of acetone and 10 ml of water with 3 ml of 1 N sodium hydroxide on a steam bath. The heating was continued to remove the acetone. Afterwards the solution was cooled to room temperature with stirring. When the cleavage was complete, the precipitate title compound was filtered and washed with water.

The yield was 330 mg. (47%) and the mass spectrum showed the expected molecular ion at m/e = 249, 172 (loss of -phenyl), 115 (representing -benzimidazole ring). pH 8.61, $pK_a$ 7.05 (66% $DMF/H_2O$).

The following elemental analysis was obtained:

Calculated for $C_{16}H_{15}N_3$:

Theory: C, 77.08; H, 6.06; N, 16,85.

Found: C, 76.82; H, 6.13; N, 16.63.

NMR (DMSO)

| δ | H's | Multiplicity | Group |
|---|---|---|---|
| 1.66 | 3 | doublet | $=CH-CH_{\underline{3}}$ |
| 6.0 | 1 | quartet | $=C\underline{H}-CH_3$ |
| 6.2 | 2 | broad singlet | $-NH_2$ |
| 6.6-7.5 | 8 | multiplet | aromatic |

Example 4

2-amino-5(6)-benzoylbenzimidazole

A slurry of 50 g of 2-amino-1-isopropylsulfonyl-5-benzoylbenzimidazole was heated in 300 ml of acetone and 500 ml of water with 350 ml of 1N sodium hydroxide on a steam bath for two hours. The heating was continued to remove the acetone. Afterwards the solution was cooled to room temperature with stirring. When the cleavage was complete, the precipitate title compound was filtered and washed with water. m/e 237, 160, 105, 77.

NMR (DMSO)

| δ | H's | Multiplicity |
|---|---|---|
| 6.8 | 3 | broad singlet |
| 7.2-8.2 | 8 | multiplet |

Example 5

2-amino-5(6)-(propanoyl)benzimidazole

A slurry of 1 g (0.003 moles) of 2-amino-1-isopropylsulfonyl-6-propanoylbenzimidazole was heated in 10 ml of water and 10 ml of acetone with 4 ml of 1 N sodium hydroxide on a steam bath for about 4 hours. After the acetone was boiled off, the solution was diluted with water and neutralized. The precipitate was extracted with ethyl acetate, then dried with magnesium sulfate, and concentrated *in vacuo*.

The yield was 320 mg/(56%) and the mass spectrum showed the expected molecular ions at m/e = 189 and 160. The NMR spectrum was consistent with the structure.

Example 6

2-amino-5(6)-(methylidenebenzyl)benzimidazole

A slurry of 5.1 g (0.015 mole) of 2-amino-1-isopropylsulfonyl-6-(methylidenebenzyl)benzimidazole was heated in 50 ml of acetone and 50 ml of water with 16 ml of 1N sodium hydroxide on a steam bath. After the acetone was boiled off, the solution was cooled to room temperature with stirring. When the cleavage was complete, the precipitate title compound was filtered and washed with water.

The yield was 3.25 g (92%) and the mass spectrum showed the expected molecular ion at m/e = 235. pH 8.98, $pK_a$ 6.8 (66% DMF/$H_2O$).

The following elemental analysis was obtained:
Calculated for $C_{15}H_{13}N_3$:

Theory: C, 76.57; H, 5.57; N, 17.86.
Found: C, 76.86; H, 5.91; N, 17.66.

NMR (DMSO)

| δ | H's | Multiplicity | Group |
|---|---|---|---|
| 5.4 | 2 | doubled doublet | $=C\underline{H}_2$ |
| 6.4 | 3 | broad singlet | |
| 6.8-7.5 | 8 | aromatic multiplet | |

Example 7

2-amino-5(6)-benzoylbenzimidazole

A slurry of 2.5 g (0.0075 moles) of 2-amino-1-isopropylsulfonyl-5(6)-benzoylbenzimidazole was heated in 25 ml of water and 25 ml of acetone with 16 ml of 1N sodium hydroxide until complete solution of

the reactants occurred. To the solution was added 0.9 ml (0.01 moles) of methyl chloroformate. The reaction mixture was then heated in a steam bath until the acetone boiled off. The resultant precipitate was filtered to provide 1.43 g of the product. pH 9.85, $pK_a$ 5.65, 13.0. m/e = 237, 160, 105, 77.

NMR (DMSO)

| δ | H's | Multiplicity |
|---|---|---|
| 6.8 | 3 | broad singlet |
| 7.2-8.2 | 8 | multiplet |

Example 8

2-amino-5(6)-benzoylbenzimidazole

The procedure of Example 7 was repeated using the same quantities of materials, but the reaction mixture was not heated and only allowed to stand at room temperature overnight (16 hours). The mixture was diluted with water and the precipitate collected to yield 1.39 g (73%) of the product. An NMR and mass spectra identical with Example 7 were obtained. pH 9.8, $pK_a$ 5.7, 13.15. (66% DMF/$H_2O$).

The following elemental analysis was obtained:
Calculated for $C_{14}H_{11}N_3O$:

Theory: C, 70.87; H, 4.67; N, 17.71.
Found: C, 71.15; H, 4.95; N, 17.74.

Example 9

2-amino-5(6)-benzoylbenzimidazole

A slurry of 5 g (0.015 moles) of 2-amino-1-isopropylsulfonyl-5(6)-benzoylbenzimidazole in 50 ml of water and 50 ml of tetrahydrofuran with 18 ml of 1N sodium hydroxide was heated on a steam bath. After a complete solution was formed, the reaction mixture was cooled to room temperature and 1N hydrochloric acid was added. The solution was concentrated under reduced pressure. The residue was titurated with methanol/water. The white solid was filtered and TLC (ethyl acetate) indicated the desired product. Yield = 2.36 g (66%). m/e 237, 160, 105, 77. pH 7.2, $pK_a$ 5.6, 13.0. (66% DMF/$H_2O$).

NMR (DMSO)

| δ | H's | Multiplicity |
|---|---|---|
| 5.6 | 2 | broad singlet |
| 6.8 | 1 | broad singlet |
| 7.2-8.0 | 8 | multiplet |

Example 10

2-amino-5(6)-benzoylbenzimidazole

A slurry of 20 g (0.058 moles) of 2-amino-1-isopropylsulfonyl-5(6)-benzoylbenzimidazole in 150 ml of water with 70 ml of 1N sodium hydroxide was heated on a steam bath. After one hour 50 ml of acetone was added and heating continued for 3 hours. The reaction mixture was held overnight (16 hours) at room temperature, then filtered, extracted 3 times with 200 ml of ethyl acetate, washed with brine, treated with carbon,

and concentrated under reduced pressure. The residue was titurated with methanol and TLC (ethyl acetate) indicated the desired product. Yield 5.2 g. m/e 237, 160, 105, 77. pH 8.59, $pK_a$ 5.41, 12.89. (66% DMF/$H_2O$).

NMR (DMSO)

| δ | H's | Multiplicity |
|---|---|---|
| 6.8 | 2 | broad singlet |
| 7.2-7.8 | 8 | multiplet |
| 11 | 1 | broad singlet |

CLAIMS

1. A process for preparing a 2-amino-5(6)-substituted-benzimidazole of the formula

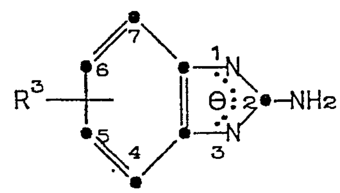

$M^+$

I

wherein:

$R^3$ is $C_1$-$C_4$ alkyl, $C_1$-$C_4$ alkoxy, halo, nitro, $C_1$-$C_4$ alkylthio, phenylthio, phenoxy, carboxy, methylsulfonyl, trifluoromethyl, $R^4-\overset{Z}{\overset{\|}{C}}-$ , or 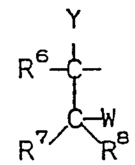 ;

$R^4$ is hydrogen, $C_1$-$C_7$ alkyl, $C_3$-$C_7$ cycloalkyl, ($C_3$-$C_7$ cycloalkyl)methyl, 1-($C_3$-$C_7$ cycloalkyl)ethyl, thienyl, benzyl, phenyl, or mono-substituted phenyl wherein said phenyl substituent is $C_1$-$C_4$ alkyl, $C_1$-$C_4$ alkoxy, chloro, bromo, iodo, nitro, or trifluoromethyl;

$R^6$ is hydrogen, $C_1$-$C_7$ alkyl, $C_3$-$C_7$ cycloalkyl, ($C_3$-$C_7$ cycloalkyl)methyl, 1-($C_3$-$C_7$ cycloalkyl)-ethyl, phenyl, or mono-substituted phenyl, wherein said phenyl substituent is $C_1$-$C_4$ alkyl, $C_1$-$C_4$ alkoxy, chloro, bromo, iodo, nitro, or trifluoromethyl;

$R^7$ and $R^8$ independently are hydrogen, halo, cyano, hydroxymethyl, nitro, $\overset{(O)_m}{S}$ - $C_1$-$C_4$ alkyl, $CH_2R^9$, $COR^9$, phenyl or mono-substituted phenyl, wherein said phenyl substituent is $C_1$-$C_4$ alkyl, $C_1$-$C_4$ alkoxy, chloro, bromo, iodo, nitro, or trifluoromethyl;

$R^9$ is hydroxy, $C_1$-$C_4$ alkoxy, halo, $C_3$-$C_6$ cycloalkyl-$C_1$-$C_4$ alkoxy, or (O-$C_1$-$C_4$ alkyl)$_p$ $NR^{10}R^{11}$ wherein

$R^{10}$ and $R^{11}$ independently are hydrogen or $C_1$-$C_4$ alkyl;

Z is oxygen, $C_1$-$C_7$ alkylidene, or $C_1$-$C_4$ alkoxyimino;

Y is hydrogen, and W is hydroxy, or together Y and W form a bond;

m is 0, 1, or 2;

p is 0 or 1;

$M^+$ is an alkali metal cation or a hydronium ion;

which comprises reacting a 1-sulfonyl-2-amino-5(6)-substituted-benzimidazole of the formula

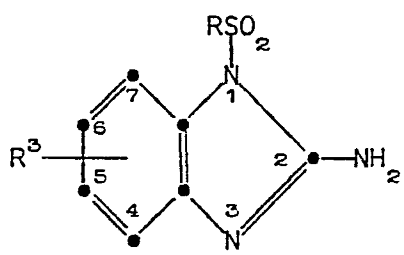

II

wherein:

$R^3$ is defined as above;

R is $C_1$-$C_5$ alkyl, $C_3$-$C_7$ cycloalkyl, phenyl, furyl, or thienyl;

with an alkali metal hydroxide or carbonate, in an inert aqueous organic solvent system, at a temperature from about 20°C to about 100°C.

2. The process of Claim 1 wherein the alkali metal hydroxide is sodium hydroxide.

3. The process of Claim 1 or 2 wherein the aqueous organic solvent system is acetone.

4. The process of Claim 1, 2 or 3 wherein the temperature is reflux.

5. The process of Claim 1 wherein $R^3$ is 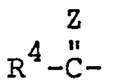 where $R^4$ is phenyl and Z is oxygen.

6. The process of Claim 1 wherein $R^3$ is 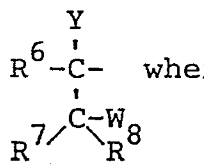 where Y and W are a bond.

7. The process of Claim 6 wherein $R^6$ is phenyl.

8. The process of Claim 1 wherein $R^3$ is 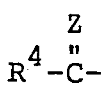 where Z is $C_1$-$C_7$ alkylidene.

9. The process of Claim 8 wherein Z is ethylidene.

10. The process of Claim 8 or 9 wherein $R^4$ is phenyl.

0091796

European Patent Office

Application number

# EUROPEAN SEARCH REPORT

EP 83301977.1

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | US - A - 4 289 782 (PAGET)<br>* Columns 1-3 *<br>-- | 1 | C 07 D 235/30<br>C 07 D 409/02//<br>(C 07 D 409/02<br>C 07 D 235/30<br>C 07 D 332/22) |
| A,P | US - A - 4 338 329 (PAGET)<br>* Columns 1-3 *<br>-- | 1 | |
| A | FR - A - 2 302 734 (JANSSEN)<br>* Formula I; page 2, lines 25-30 *<br>-- | 1 | |
| A | US - A - 3 655 901 (JENSEN)<br>* Column 2, lines 45-70 *<br>-- | 1 | |
| D,A | US - A - 4 118 742 (PAGET)<br>* Column 2, lines 19-32 *<br>-- | 1 | TECHNICAL FIELDS SEARCHED (Int. Cl. 3) |
| D,A | US - A - 4 174 454 (PAGET)<br>* Column 2, lines 28-40 *<br>-- | 1 | C 07 D 235/00<br>C 07 D 409/00 |
| D,A | US - A - 4 018 790 (PAGET)<br>* Column 2, lines 19,20 *<br>-- | 1 | |
| D,A | US - A - 4 118 573 (PAGET)<br>* Column 2, lines 11-22 *<br>-- | 1 | |
| D,A | US - A - 4 008 243 (WIKEL)<br>* Column 2, formula III *<br>-- | 1 | |

The present search report has been drawn up for all claims

| Place of search | Date of completion of the search | Examiner |
|---|---|---|
| VIENNA | 27-06-1983 | BRUS |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503. 03.82

0091796

European Patent Office

# EUROPEAN SEARCH REPORT

Application number

EP 83301977.1

| DOCUMENTS CONSIDERED TO BE RELEVANT | | | CLASSIFICATION OF THE APPLICATION (Int. Cl.³) |
|---|---|---|---|
| Category | Citation of document with indication, where appropriate, of relevant passages | Relevant to claim | |
| D,A | US - A - 4 150 028 (PAGE)<br>* Column 3, lines 26-40 *<br>---- | 1 | |
| | | | TECHNICAL FIELDS SEARCHED (Int. Cl.³) |